# EUROPEAN PATENT APPLICATION

(11) **EP 4 570 286 A1**
(43) Date of publication of application: **18.06.2025**
(21) Application number: 23913863.9
(22) Date of filing: 13.10.2023
(51) Int. Cl.: A61M 5/32

(54) **INSULIN NEEDLE TIP PROTECTION MECHANISM AND INSULIN INJECTION DEVICE**

(71) Applicant: Ningbo Medsun Medical Co., Ltd., Ningbo, Zhejiang 315034 (CN)
(72) Inventor: SHI, Lei, Ningbo, Zhejiang 315034 (CN); CHEN, Zhicheng, Ningbo, Zhejiang 315034 (CN)
(74) Representative: Ipside
(86) International application number: PCT/CN2023/124618
(87) International publication number: WO 2025/076843

(57) **Abstract**

This invention discloses a protective mechanism for insulin needles and an insulin injection device. Through the cooperation of a U-shaped reciprocating slot and a flexible wall, it is possible to automatically retract the needle tip after injection, and prevent it from being pushed out again, thus achieving the purpose of needle tip protection and avoiding reuse.

## Description

### Technical Field

This invention relates to an insulin injection device, particularly to an insulin needle tip protection mechanism.

### Background of the Invention

Insulin injection devices, such as insulin pens and syringes, as a method for treating diabetes, have been widely used. Their main function is to accurately inject insulin into the body of diabetic patients to control their blood sugar levels. Moreover, as the number of diabetic patients continues to grow, the demand for insulin injection devices is increasing.

After insulin injection, if the needle is left exposed, it can easily lead to accidental injuries, which may cause cross-infection. At the same time, due to the lack of necessary protective structures, such needles can be reused after use, posing a very high risk of infection.

Therefore, technicians in this field are committed to developing an insulin needle tip protection mechanism.

### Summary of the Invention

In order to achieve the above objective, the present invention first provides an insulin needle tip protection mechanism, it comprises a needle holder, a spring, and a tail seal member; the needle holder has a cylinder, and the tail seal member is installed inside the cylinder equipped with the spring; the head of the tail seal member has a pin head, and its side wall has a flexible wall; the flexible wall protrudes outward from the main body of the tail seal member which can be pressed inward towards the interior of the tail seal member; the side wall of the cylinder of the needle holder has a U-shaped reciprocating slot; the pin head of the tail seal member is located within the U-shaped reciprocating slot and can move up and down within it; at the bottom of the cylinder of the tail seal member, there is an oblique keyhole slot, when the flexible wall of the tail seal member aligns with the oblique keyhole slot in a circumferential direction, then pushing the tail seal member, the flexible wall can be pressed into the tail seal member through the oblique keyhole slot; when the flexible wall of the tail seal member is misaligned with the oblique keyhole slot in a circumferential direction, it cannot be pushed into the cylinder due to the obstruction by the flexible wall; there is a beveled edge set at the bend of the U-shaped reciprocating slot, making one groove of the U-shaped reciprocating slot longer and the other shorter; the insulin needle tip protection mechanism is assembled in such a way that before injection, the pin head of the tail seal member is located at the bottom of the short groove of the U-shaped reciprocating slot, and at this time, the flexible wall of the tail seal member is aligned with the oblique keyhole slot in a circumferential direction; when the insulin pen is screwed or the insulin needle is pushed, the tail seal member can compress the spring and advance towards the inside of the cylinder, and the pin head also moves upward within the short slot, when it reaches the bend of the U-shaped reciprocating slot, it diagonally moves under the action of the beveled edge to the top of the long groove of the U-shaped reciprocating slot; when the injection is completed and the external force is removed, the tail seal member and its pin head retract backward under the restoring force of the spring, until the pin head moves downward to the bottom of the long groove of the U-shaped reciprocating slot, at the same time, the flexible wall of the tail seal member exits the oblique keyhole slot and returns to its protruding state, and because the tail seal member as a whole has rotated during the advancement process, at this time, the flexible wall of the tail seal member is misaligned with the oblique keyhole slot in a circumferential direction, so even if the tail seal member is pushed again, it cannot advance to the injection position and exposing the needle tip again.

Further, the tail seal member includes two symmetrical flexible walls and the inner ends of the left and right flexible walls are respectively provided with semicircular clamping portions, the semicircular clamping portions have an angle when the flexible walls are in an unfolded state, the angle is set to become vertical when the flexible walls are pressed into place, so that when the needle tip is exposed, the needle is clamped tightly at the same time.

Further, the oblique keyhole slot is a cross-shaped slot.

Further, the tail seal member includes two symmetrical flexible walls.

Further, the tail seal member includes two symmetrical pin heads.

Further, the flexible walls and pin heads of the tail seal member are spaced 90° apart in a circumferential direction.

Further, at the top of the short slot of the U-shaped reciprocating slot, near the bend of the slot, there is an interference section, the width D of the interference section is slightly less than the diameter of the pin head, thus creating an interference fit with respect to the pin head.

Further, the tail seal member is formed as a single integrated piece by injection molding.

This invention also provides an insulin injection device that includes the aforementioned insulin needle tip protection mechanism.

Further, he insulin injection device is an insulin pen or an insulin syringe.

The insulin needle tip protection mechanism provided by this invention allows the needle tip to automatically retract after injection and cannot be pushed out again. This achieves the purpose of protecting the needle tip and also prevents the needle from being used a second time.

The following will further explain the concept, specific structure, and technical effects of the present invention in conjunction with the drawings, to fully understand the purpose, characteristics, and effects of the invention.

### Brief description of the Drawings

Figure 1 is a three-dimensional diagram of the insulin needle tip protection mechanism in a preferred embodiment of the present invention.
Figure 2 is an exploded view diagram of the insulin needle tip protection mechanism in a preferred embodiment of the present invention.
Figure 3 is a three-dimensional diagram of the tail seal member in a preferred embodiment of the present invention.
Figure 4 is a partial cross-sectional view of the needle holder in a preferred embodiment of the present invention.
Figure 5 is a partial cross-sectional view of the needle holder and tail seal member in a preferred embodiment of the present invention.
Figure 6 is a front view when the pin head is located at the bottom of the short slot in a preferred embodiment of the present invention.
Figure 7 is a cross-sectional view of Figure 6.
Figure 8 is a front view when the pin head is located at the beveled edge of the bend in a preferred embodiment of the present invention.
Figure 9 is a front view when the pin head is located at the top of the long slot in a preferred embodiment of the present invention.
Figure 10 is a front view when the pin head is located at the bottom of the long slot in a preferred embodiment of the present invention.
Figure 11 is a cross-sectional view of Figure 10.
Figure 12 is a cross-sectional view of the tail seal member in a preferred embodiment of the present invention.
Figure 13 is a cross-sectional view of Figure 12.
Figure 14 is a schematic diagram of the U-shaped reciprocating slot with an interference section in a further embodiment of the present invention.

### Detailed Description of Embodiments

A plurality of preferred embodiments of the present invention are described below with reference to the drawings, which makes its technical content more clear and convenient to understand. The present invention may be embodied in many different forms of embodiments, and the scope of protection of the present invention is not limited to the embodiments set forth herein.

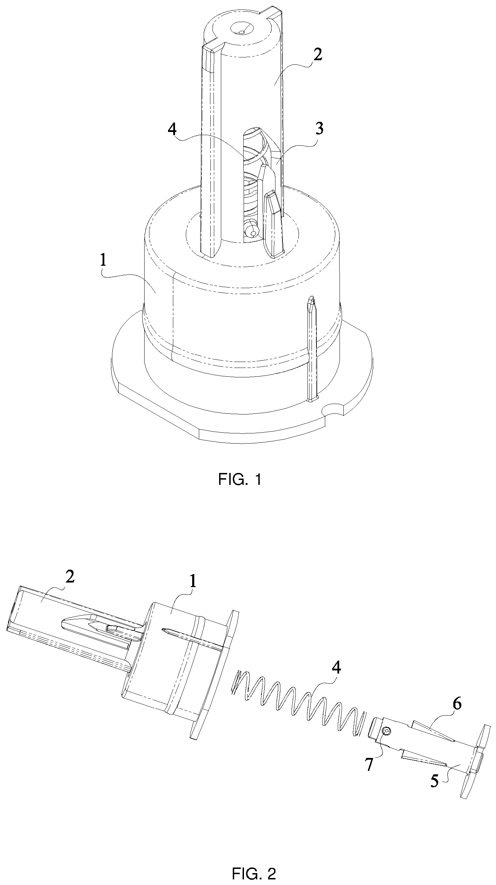

As shown in Figures 1 and 2, the insulin needle tip protection mechanism according to the present invention includes a needle holder 1, spring 4, and tail seal member 5, which are assembled together. The needle holder 1 has a cylinder 2, and the tail seal member 5 is installed inside the cylinder 2 equipped with the spring 4.

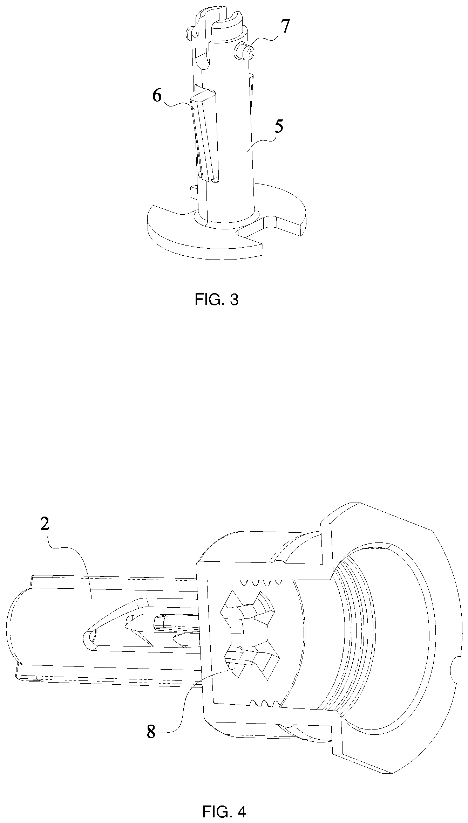

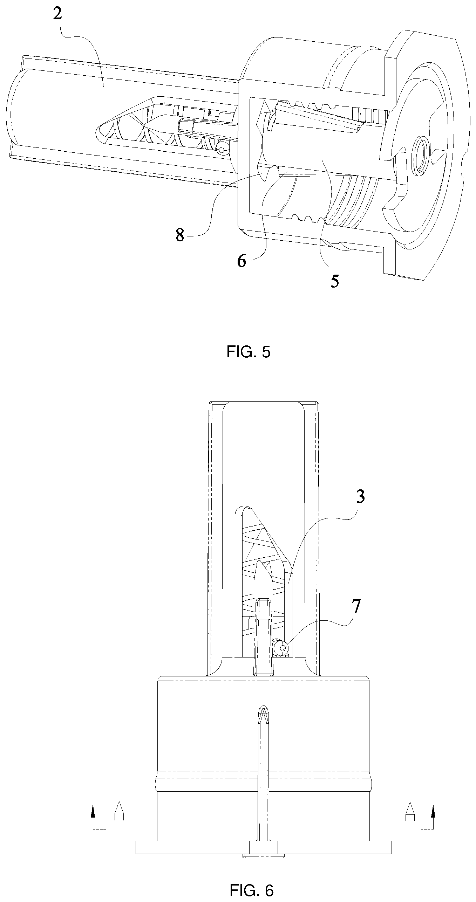

Refer also to Figures 3 and 4, the tail seal member 5 has a pin head 7 at its head, and a flexible wall 6 on its side wall. The flexible wall 6 protrudes outward from the main body of the tail seal member, but can be pressed inward towards the interior of the tail seal member 5. The side wall of the cylinder 2 of the needle holder 1 is provided with a U-shaped reciprocating slot 3. After assembly, the pin head 7 of the tail seal member 5 is located within the U-shaped reciprocating slot 3 and can move up and down within the U-shaped reciprocating slot 3. At the bottom of the cylinder 2 of the tail seal member 5, there is a keyhole slot 8. When the flexible wall 6 of the tail seal member 5 aligns with the keyhole slot 8 in a circumferential direction, then pushing the tail seal member 5, the flexible wall 6 is pressed into the tail seal member 5 through the keyhole slot 8, while the tail seal member 5 compresses the spring 4 and advances towards the inside of the cylinder 2. When the flexible wall 6 of the tail seal member 5 is misaligned with the keyhole slot 8 in a circumferential direction, the flexible wall 6 is blocked and cannot advance towards the inside of the cylinder 2, as shown in Figure 5.

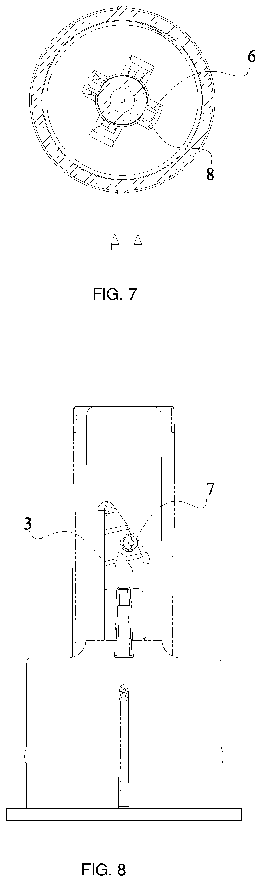

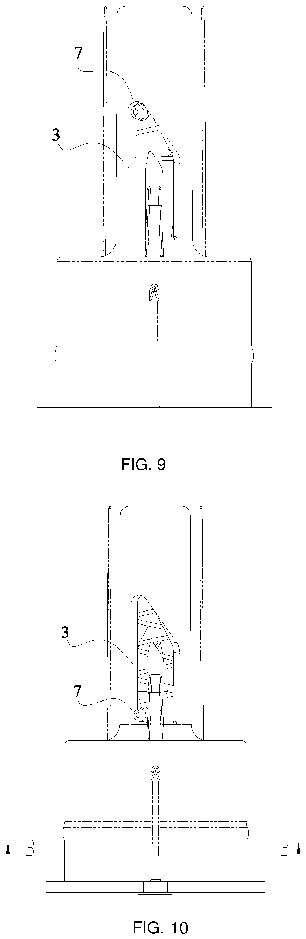

As shown in Figure 6, there is a beveled edge arranged at the bend of the U-shaped reciprocating slot 3, which results in one groove being longer and the other shorter within the U-shaped reciprocating slot 3. Before injection, the pin head 7 of the tail seal member 5 is located at the bottom of the short groove of the U-shaped reciprocating slot 3, and at this time, as shown in Figure 7, the flexible wall 6 of the tail seal member 5 is aligned with the keyhole slot 8. When the insulin pen is screwed or the insulin needle is pushed, the tail seal member 5 can compress spring 4 and advance towards the inside of the cylinder 2, while the pin head 7 also moves upward within the short groove. When it reaches the bend of the U-shaped reciprocating slot 3, it diagonally moves due to the restriction of the beveled edge, reaching the top of the long groove of the U-shaped reciprocating slot 3, as shown in Figures 8 and 9. After the injection is completed and the external force is removed, the tail seal member 5 and its pin head 7 retract backward under the restoring force of spring 4 until the pin head 7 moves downward to the bottom of the long groove of the U-shaped reciprocating slot 3. At the same time, the flexible wall 6 of the tail seal member 5 exits the keyhole slot 8, returning to its protruding state. Since the tail seal member 5 as a whole has rotated during the advancement process, at this point, the flexible wall 6 of the tail seal member 5 is misaligned with the keyhole slot 8 in a circumferential direction. Therefore, even if the tail seal member 5 is pushed again, it cannot advance to the injection position and expose the needle tip.

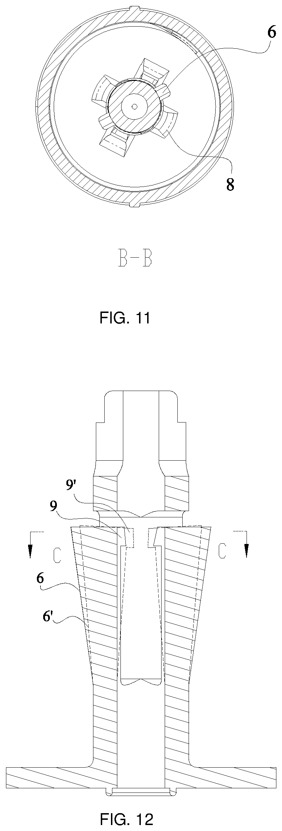

As shown in Figures 12-13, in a further embodiment of the present invention, the tail seal member 5 includes two symmetrical flexible walls 6, and the inner ends of the left and right flexible walls 6 are respectively provided with semicircular clamping portions 9. The semicircular clamping portions 9 have an angle when the flexible walls 6 are in an unfolded state. This angle is set to become vertical when the flexible walls 6 are pressed into place. , so that when the needle tip is exposed, the needle 10 is clamped tightly at the same time, so that the needle can be kept stable during injection.

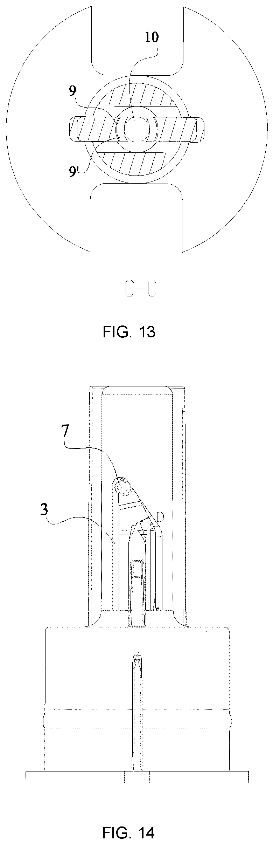

As shown in Figure 14, in another advanced embodiment of the invention, there is an interference section located at the top of the short slot of the U-shaped reciprocating slot 3, near the bend of the slot. The width D of the interference section is slightly less than the diameter of the pin head 7, creating an interference fit with respect to pin head 7. This means that when the tail seal member 5 is injected, the pin head 7 must "squeeze past" this interference section. Consequently, when the tail seal member 5 retracts under the restoring force of spring 4, the pin head 7 will not re-enter the short slot due to any disoperation by the user.

Preferred embodiments of the present invention are described in detail above. It should be understood that many modifications and variations can be made to the concepts of the present invention without creative efforts by those of ordinary skill in the art. Therefore, it would be obvious to a person skilled in the art to arrive at the technical solutions of the present invention by means of logical analysis, reasoning, or limited experiments on the basis of the prior art, all within the scope of protection defined by the claims.

## Claims

1. An insulin needle tip protection mechanism, **characterized in that**:
it comprises a needle holder, a spring, and a tail seal member; the needle holder has a cylinder, and the tail seal member is installed inside the cylinder equipped with the spring; the head of the tail seal member has a pin head, and its side wall has a flexible wall; the flexible wall protrudes outward from the main body of the tail seal member which can be pressed inward towards the interior of the tail seal member; the side wall of the cylinder of the needle holder has a U-shaped reciprocating slot; the pin head of the tail seal member is located within the U-shaped reciprocating slot and can move up and down within it; at the bottom of the cylinder of the tail seal member, there is an oblique keyhole slot, when the flexible wall of the tail seal member aligns with the oblique keyhole slot in a circumferential direction, then pushing the tail seal member, the flexible wall can be pressed into the tail seal member through the oblique keyhole slot; when the flexible wall of the tail seal member is misaligned with the oblique keyhole slot in a circumferential direction, it cannot be pushed into the cylinder due to the obstruction by the flexible wall;
there is a beveled edge set at the bend of the U-shaped reciprocating slot, making one groove of the U-shaped reciprocating slot longer and the other shorter;
the insulin needle tip protection mechanism is assembled in such a way that before injection, the pin head of the tail seal member is located at the bottom of the short groove of the U-shaped reciprocating slot, and at this time, the flexible wall of the tail seal member is aligned with the oblique keyhole slot in a circumferential direction; when the insulin pen is screwed or the insulin needle is pushed, the tail seal member can compress the spring and advance towards the inside of the cylinder, and the pin head also moves upward within the short slot, when it reaches the bend of the U-shaped reciprocating slot, it diagonally moves under the action of the beveled edge to the top of the long groove of the U-shaped reciprocating slot; when the injection is completed and the external force is removed, the tail seal member and its pin head retract backward under the restoring force of the spring, until the pin head moves downward to the bottom of the long groove of the U-shaped reciprocating slot, at the same time, the flexible wall of the tail seal member exits the oblique keyhole slot and returns to its protruding state, and because the tail seal member as a whole has rotated during the advancement process, at this time, the flexible wall of the tail seal member is misaligned with the oblique keyhole slot in a circumferential direction, so even if the tail seal member is pushed again, it cannot advance to the injection position and exposing the needle tip again.

2. The insulin needle tip protection mechanism according to claim 1, wherein the tail seal member includes two symmetrical flexible walls and the inner ends of the left and right flexible walls are respectively provided with semicircular clamping portions , the semicircular clamping portions have an angle when the flexible walls are in an unfolded state, the angle is set to become vertical when the flexible walls are pressed into place, so that when the needle tip is exposed, the needle is clamped tightly at the same time.

3. The insulin needle tip protection mechanism according to claim 1, wherein the oblique keyhole slot is a cross-shaped slot.

4. The insulin needle tip protection mechanism according to claim 1, wherein the tail seal member includes two symmetrical flexible walls.

5. The insulin needle tip protection mechanism according to claim 1, wherein the tail seal member includes two symmetrical pin heads.

6. The insulin needle tip protection mechanism according to claim 1, wherein the flexible walls and pin heads of the tail seal member are spaced 90° apart in a circumferential direction.

7. The insulin needle tip protection mechanism according to claim 1, wherein at the top of the short slot of the U-shaped reciprocating slot, near the bend of the slot, there is an interference section, the width D of the interference section is slightly less than the diameter of the pin head, thus creating an interference fit with respect to the pin head.

8. The insulin needle tip protection mechanism according to claim 1, wherein the tail seal member is formed as a single integrated piece by injection molding.

9. An insulin injection device, **characterized in that** it includes the insulin needle tip protection mechanism according to claims 1-8.

10. The insulin injection device according to claim 9, wherein the insulin injection device is an insulin pen or an insulin syringe.
